# FASCICULE DE BREVET EUROPEEN

(11) **EP 1 404 327 B1**
(45) Date de publication et mention de la délivrance du brevet: **28.06.2006**
(21) Numéro de dépôt: 02732833.5
(22) Date de dépôt: 17.04.2002
(51) Int. Cl.: A61K 31/455, A61K 31/133, A61P 17/00

(54) **COMPOSITION DERMATOLOGIQUE COMPRENANT L'ACIDE NICOTINIQUE OU UN AMIDE, ET UNE BASE SPHINGOÏDE.**
DERMATOLOGISCHE ZUSAMMENSETZUNGEN, DIE NIKOTINSÄURE ODER NIKOTINSÄUREAMID UND SPHINGOID-BASE ENTHALTEN
DERMATOLOGICAL COMPOSITION COMPRISING NICOTINIC ACID OR AN AMIDE, AND A SPHINGOID BASE

(30) Priorité: 23.04.2001 FR 0105451
(43) Date de publication de la demande: 07.04.2004
(73) Titulaire: Dermaconcept JMC, 80000 Amiens (FR)
(72) Inventeur: ALLART, Jean-Claude, F-62700 Bruay Labuissiere (FR); LEFEVRE, Jean-Marie, F-80000 Amiens (FR); PEYROT, Jacques, F-63000 Clermont Ferrand (FR)
(74) Mandataire: L'Helgoualch, Jean
(86) Numéro de dépôt international: PCT/FR2002/001330
(87) Numéro de publication internationale: WO 2002/085362

(56) Documents cités:
- EP-A- 0 052 705
- EP-A- 0 919 226
- WO-A-99/47114
- DE-A- 19 810 999
- DATABASE WPI Section Ch, Week 199711 Derwent Publications Ltd., London, GB; Class B02, AN 1997-115199 XP002185450 & JP 09 002952 A (KANEBO LTD), 7 janvier 1997 (1997-01-07)
- DATABASE WPI Section Ch, Week 200037 Derwent Publications Ltd., London, GB; Class B05, AN 2000-425564 XP002185449 & JP 2000 109409 A (KAO CORP), 18 avril 2000 (2000-04-18)

## Description

La présente invention concerne une composition comprenant le nicotinamide et au moins une base sphingoïde choisie parmi la phytosphingosine, la tétracétylphytosphingosine, la N-acétylphytosphingosine et le chlorhydrate de phytosphingosine, pour le traitement des troubles de la kératinisation en dermatologie humaine ou animale.

La peau est un véritable organe doté de propriétés essentielles à la vie. Elle comprend des couches superficielles, à savoir l'épiderme, et des couches plus profondes, le derme et l'hypoderme, et chacune possède des propriétés spécifiques permettant à l'ensemble de réagir et s'adapter aux conditions de son environnement. Elle joue un rôle de protection vis à vis de l'environnement, ainsi qu'un rôle immunitaire dont le bon fonctionnement conditionne le bien-être de l'individu. Tout trouble de sa physiologie a des conséquences pathologiques de gravité variable, mais justifiant toujours des soins rapides et adaptés. C'est dire l'importance de bien comprendre les mécanismes qui interviennent de façon à pouvoir apporter une réponse thérapeutique judicieuse, ne risquant pas d'aggraver la situation.

L'acné et la dermite atopique sont des affections de l'épiderme affectant un grand nombre d'individus, y compris les enfants et les adolescents. La dermite atopique est une affection fréquente touchant les individus des deux sexes dès l'âge de trois mois, se caractérisant par des poussées d'eczéma répétitives sur une peau présentant des anomalies constitutionnelles, tandis que l'acné est une affection cutanée liée à des anomalies des glandes sébacées, touchant principalement les adolescents et les adultes jeunes.

L'acné résulte de l'occlusion de l'extrémité supérieure ainsi que de la partie interne du canal pilo-sébacé par la multiplication anormale des kératinocytes, et de l'hyperactivité hormonale androgénique apparaissant souvent lors de la puberté, qui provoque une importante augmentation de la séborrhée au niveau des glandes sébacées. L'obstruction du canal pilo-sébacé provoque la formation de comédons ou microkystes, s'accompagnant d'une prolifération de bactéries Propionibacterium acnes ainsi que de Pytirosporum ovale dans les follicules pilo-sébacés obstrués.

Cette affection, particulièrement fréquente chez les adolescents, s'accompagne d'une réaction inflammatoire de la peau, pouvant se présenter sous forme de papules ou pustules généralement localisées au niveau du derme superficiel. Dans certains cas la réaction inflammatoire peut atteindre le derme profond, formant des nodules et macrokystes.

Les traitements classiques de l'acné utilisent le peroxyde de benzoyle, l'érythromycine, l'isotrétinoïne et divers antiseptiques. Cependant, l'utilisation d'antibiotiques tels que l'érythromycine se heurte aujourd'hui à des phénomènes de résistance bactérienne et des modifications de flore, tandis que le peroxyde de benzoyle entraîne des irritations et des allergies cutanées.

La dermite atopique est une inflammation chronique de la peau généralement liée à un trouble originel de la kératinisation, à la présence de surpopulation bactérienne (Staphylococcus aureus) ou levurique (Pytirosporum ovale) à la surface cutanée, dont le rôle immunogène explique l'existence d'un état inflammatoire latent, ou encore à des anomalies des réactions immunitaires liées à une pénétration accrue d'allergènes potentiels de l'environnement et une réponse lymphocytaire perturbée.

Les traitements généralement proposés pour la dermite atopique ont pour but d'agir sur les divers paramètres qui déclenchent cette affection. Ainsi l'utilisation d'antiseptiques pour réduire la surpopulation bactérienne est de plus en plus souvent contestée dans le milieu médical car elle fragilise la peau et sélectionne certains germes, les émollients traditionnels sont parfois mal tolérés par les patients, les antihistaminiques sont généralement inefficaces, les corticoïdes appliqués localement ont pour inconvénient d'induire une atrophie dermo-épidermique et un risque d'effet systémique, et la cyclosporine peut provoquer une insuffisance rénale. Des études récentes ont montré que certains agents modulateurs de la réponse immunitaire à activité anti-inflammatoire tels que l'ascomycine et des dérivés pourraient être utilisés dans le traitement de la dermite atopique [K. Rappersberger et al., "Clearing of psoriasis by a novel immunosuppressive macrolide" J. Invest. Dermatol. (1996) 106, 701-710].

Les propriétés de l'acide nicotinique et du nicotinamide sont bien connues en thérapeutique et en dermatologie. Ces vitamines sont par exemple utiles pour le traitement de l'acné en particulier acne vulgaris, et le brevet EP-B-052.705 décrit diverses formes liquides et solides destinées à son utilisation dans ces indications. Cependant, on sait que l'acide nicotinique et le nicotinamide (vitamine PP) utilisés isolément, n'offrent pas une efficacité thérapeutique satisfaisante dans le traitement de l'acné ou de la dermite atopique.

Le brevet FR-A-2.780.888 décrit une lotion destinée au traitement dermatologique des comédons, comprenant essentiellement une association de nicotinamide (vitamine PP) et d'hydroxy-acides, plus particulièrement d'acide mandélique. Quelques dérivés d'acide nicotinique, et en particulier la vitamine B3, ont été proposés en association avec des précurseurs de céramides dans la demande WO 99.47114 mais les compositions les contenant sont destinées à l'hydratation de peaux normales et non à des traitements dermatologiques de peaux pathologiques.

On sait que des bases sphingoïdes telles que la phytosphingosine et la sphingosine, précurseurs de céramides, sont présentes dans la peau humaine, et des études ont montré que ces molécules ont des propriétés inhibitrices de la protéine kinase C, et semblent impliquées dans la différenciation des kératinocytes de l'épiderme. On a également observé que des sphingosines présentes dans le stratum corneum et d'autres couches de l'épiderme, inhibent la croissance de certains micro-organismes indésirables.

Diverses publications décrivent l'utilisation de la sphingosine et de la phytosphingosine dans des compositions cosmétiques et dermatologiques. Par exemple, la demande de brevet WO 95.03028 décrit la sphingosine, utilisée dans une composition à pH voisin de 5, pour réduire l'effet irritant de certains alpha-hydroxy-acides. Le brevet EP 940.140 décrit une composition cosmétique associant des alpha-hydroxy-acides et des céramides ou des précurseurs de céramides tels que la phytosphingosine. Le document DE 19810999 décrit l'utilisation de la sphingosine, de sphingosine-1-phosphates et de dérivés pour la préparation de formulations pour le traitement d'inflammations de la peau telles que le psoriasis vulgaris, l'acné vulgaris et l'héliodermite.

Il subsiste donc un besoin de compositions dermatologiques à activité anti-inflammatoire permettant un traitement local de l'acné et de la dermite atopique dans de bonnes conditions d'efficacité et de confort du patient, tout en évitant les effets secondaires et les inconvénients des traitements classiques.

Les études effectuées par la demanderesse ont montré qu'il était possible de traiter efficacement l'acné et la dermite atopique en associant le nicotinamide (vitamine PP), et une base sphingoïde choisie parmi la phytosphingosine, la tétracétylphytosphingosine, la N-acétylphytosphingosine, et le chlorhydrate de phytosphingosine au sein de la même composition.

La présente invention a donc pour objet une nouvelle composition utile en dermatologie humaine et animale pour le traitement de l'acné, notamment l'acné vulgaris, et de la dermite atopique.

L'invention a également pour objet une composition associant le nicotinamide (vitamine PP), et une base sphingoïde pour le traitement de l'acné et de la dermite atopique.

Enfin, la présente invention a pour objet l'utilisation de nicotinamide (vitamine PP), et d'une base sphingoïde pour la préparation d'un médicament pour le traitement de l'acné et de la dermite atopique.

La composition conforme à la présente invention comprend en combinaison le nicotinamide et au moins une base sphingoïde choisie parmi la phytosphingosine, la tétracétylphytosphingosine, la N-acétylphytosphingosine, et le chlorhydrate de phytosphingosine. Suivant l'invention, la base sphingoïde préférée est la phytosphingosine ou le chlorhydrate de phytosphingosine.

le nicotinamide, est utilisé dans la composition à raison de 0,1 à 15 % en poids par rapport au poids total de la composition, et de préférence de 1 à 10 %. La teneur en base sphingoïde peut varier selon la base utilisée, mais elle est généralement comprise entre 0,01 % et 5 %, de préférence entre 0,05 et 2 %.

Suivant une forme avantageuse de réalisation, la composition de l'invention comprend en outre du ciclopirox ou de la ciclopiroxolamine, dont l'action complète efficacement celle des deux autres composants précités, c'est-à-dire la base sphingoïde et le nicotinamide.

Le ciclopirox et la ciclopiroxolamine peuvent être incorporés dans la composition à raison de 0 à 5 % en poids par rapport au poids total de la composition, et de préférence entre 0,5 % et 2 % en poids.

La vitamine PP, ainsi que les bases sphingoïdes utilisées dans les compositions de la présente invention sont généralement disponibles dans le commerce et peuvent être obtenues par des méthodes connues à partir de diverses sources appropriées.

Par exemple les bases sphingoïdes peuvent être obtenues à partir de sources naturelles ou par synthèse chimique ou par fermentation. Elles peuvent aussi être obtenues à partir de tissus animaux ou végétaux par extraction et purification. De préférence, les bases sphingoïdes utilisées dans l'invention sont préparées par fermentation microbienne, par exemple à partir d'une levure telle que Pichia ciferii, et la phytosphingosine obtenue de la sorte présente l'avantage d'être très proche de celle de la peau humaine ou animale. Suivant une forme préférentielle de réalisation de l'invention, on utilise comme base sphingoïde la phytosphingosine obtenue à partir de tétra-acétyl-phytosphingosine par désacétylation. La réaction de désacétylation peut s'effectuer par réaction chimique, par exemple par hydrolyse en milieu basique, ou par réaction enzymatique.

Les essais effectués sur des compositions conformes à la présente invention ont mis en évidence une synergie d'action tout particulièrement entre la vitamine PP et la phytosphingosine, permettant d'agir simultanément sur plusieurs paramètres et procurant une potentialisation de l'activité anti-acnéique tout en supprimant le risque de résistance bactérienne et de phénomènes irritatifs ou allergiques. Une seule composition suivant la présente invention peut ainsi être substituée à l'utilisation combinée d'un émollient, d'un antiseptique et d'un anti-inflammatoire local pour le traitement de la dermite atopique et de l'acné, ce qui a notamment pour avantage d'éviter les effets secondaires de chacun de ces produits usuels de traitement.

Le ciclopirox (dénomination commune internationale) ainsi que certains de ses sels tels que la ciclopiroxolamine, sont des composés connus pour leurs propriétés antifongiques et antibactériennes permettant leur utilisation, par exemple, dans des lotions et shampooings antipelliculaires. Par contre, leur utilisation pour le traitement de troubles de la kératinisation tels que l'acné et la dermite atopique n'avait pas été envisagée jusqu'à présent.

L'addition de ciclopirox ou d'un sel tel que la ciclopiroxolamine a pour effet d'augmenter et d'accélérer le phénomène de lyse de souches levuriques, en particulier Malassezia furfur, tandis que la base sphingoïde, notamment la phytosphingosine, exerce une action anti-inflammatoire qui limite la réaction cytokinique induite par la lyse brutale que pourrait déclencher le ciclopirox. De plus, l'acide nicotinique ou ses amides, notamment la vitamine PP, favorise la formation de céramides dans l'épiderme. Ainsi ces trois catégories de principes actifs combinent efficacement leurs activités dans la composition suivant l'invention.

Lorsqu'une composition suivant l'invention est utilisée en association avec une ou plusieurs cyclines ou l'isotrétinoïne administrées par voie orale, elle présente l'avantage de corriger les sélections de germes que peuvent induire ces médicaments, notamment les folliculites gram-négatif et les folliculites staphylococciques.

Elle permet en outre de régulariser le film lipidique de surface des peaux acnéiques par stimulation enzymatique de la sérine palmitoyl transférase et des céramidases, et par activation de la synthèse des acides gras et du cholestérol. Elle agit donc plus efficacement à la fois sur les germes de l'acné et sur l'inflammation qu'ils provoquent tout en participant à la restauration des lipides de surface dont les anomalies sont un des facteurs déclenchant l'affection.

Les excipients et supports utilisables dans les compositions conformes à la présente invention sont ceux couramment utilisés dans les préparations à usage dermatologique, et choisis en fonction de la forme d'administration retenue. A titre d'exemple, on peut citer des gélifiants, des émulsionnants, des épaississants, des conservateurs, des adoucissants, ainsi que des bases lavantes et des parfums.

On peut choisir l'agent émulsionnant parmi des polymères carboxyvinyliques à haut poids moléculaire tels que le Carbopol® , des polysorbates tels que ceux commercialisés sous les marques Tween 20® ou Tween 60® , des esters de sorbitan et par exemple un stéarate, un palmitate, un oléate ou un laurate de sorbitan (par exemple l'Arlacel® ). On peut encore utiliser comme agents émulsionnants divers dérivés d'acide stéarique ou palmitique tels que le stéarate de glycérol, un stéarate de propylène glycol, un stéarate de polyéthylène glycol, le stéarate de PEG 100®, un stéareth ou un cétéareth, ou encore le polyglycéryl-2-sesquioléate, l'éther cétylique de polyoxyéthylène, un polyglucoside de siloxane, ou une silicone émulsionnable.

Les gélifiants et épaississants sont incorporés dans la composition pour en améliorer la fluidité. Ils peuvent être choisis par exemple parmi les polyacrylamides du type Carbopol, les copolymères acrylate/acide acrylique et par exemple l'Aculyn®, les dérivés de cellulose comme l'hydroxypropyl cellulose et par exemple le Klucel®, des muco-polysaccharides végétaux, les cires comme la cire d'abeille, les argiles ou les gommes naturelles comme la gomme de xanthane.

Les adoucissants peuvent être choisis parmi les alcools gras ou les esters, et par exemple les produits à base d'alcool isostéarylique ou de sorbitol polysaccharidique commercialisés sous les marques Soothex® et Rhamnosoft®. D'une manière générale, les adoucissants usuels de la technique peuvent convenir dans l'invention.

La base lavante est généralement constituée par des tensioactifs tels que les tensioactifs ioniques, non ioniques, cationiques et anioniques, comme les bétaînes, les esters de sorbitan, et plus particulièrement le sodium laureth sulfate ou le distéarate de glycol. On peut utiliser par exemple les produits disponibles dans le commerce sous les marques Texapon® et Sinnoflor®.

Les compositions selon la présente invention peuvent se présenter sous toutes les formes galéniques usuelles adaptées à l'indication dermatologique ou cosmétologique, pour application topique.

Elles peuvent se présenter par exemple sous forme de solutions aqueuses, huileuses ou hydro-alcooliques, de dispersions, de sérums, de gels (aqueux, anhydres ou lipophiles), de lotions micellaires, de lotion hydro-alcoolique, de solutions pour pulvérisation, de suspensions, de dispersions vésiculaires ioniques ou non ioniques, d'émulsions liquides ou semi-liquides (par exemple un lait), solide ou semi-solide. Les émulsions peuvent être du type huile dans eau (H/E) ou eau dans huile (E/H), par exemple des gels, des crèmes ou des shampooings.

Pour le traitement de la dermite atopique et de l'acné, les compositions conformes à l'invention sont utilisées suivant une posologie déterminée en fonction de la gravité de l'affection et de l'état du patient. D'une manière générale, elles sont appliquées 1 à 4 fois par jour pendant une période allant de quelques jours à quelques semaines. Dans la plupart des cas, une application 2 fois par jour pendant 4 à 5 semaines est suffisante pour un traitement d'attaque, et les résultats sont déjà perceptibles dès les premiers jours.

Les exemples suivants illustrent l'invention plus en détail. Sauf indication contraire, les parties et pourcentages sont exprimés en poids.

### Exemple 1

Suivant les techniques usuelles, on prépare une lotion alcoolique pour application topique ayant la composition pondérale indiquée ci-après.

| | | |
|---|---|---|
| Chlorhydrate de phytosphingosine | | 0,30 |
| nicotinamide (vitamine PP) | | 4,00 |
| cyclométhicone | | 12,00 |
| C12-13 alkyl lactate | | 14,00 |
| éthoxy diglycol | | 6,00 |
| éthanol à 96° | q.s.p. | 100,00 |

### Exemple 2

Suivant les techniques usuelles, on prépare un gel hydroalcoolique pour application topique ayant la composition pondérale indiquée ci-après.

| | | |
|---|---|---|
| Chlorhydrate de phytosphingosine | | 0,15 |
| nicotinamide (vitamine PP) | | 4,00 |
| cyclométhicone | | 12,00 |
| C12-13 alkyl lactate | | 14,00 |
| éthoxy diglycol | | 6,00 |
| hydroxy propyl cellulose | | 1,00 |
| éthanol à 96° | q.s.p. | 100,00 |

### Exemple 3

Suivant les techniques usuelles, on prépare une lotion micellaire ayant la composition pondérale indiquée ci-après.

| | | |
|---|---|---|
| phytosphingosine | | 0,20 |
| nicotinamide (vitamine PP) | | 2,00 |
| Tween 80 | | 5,00 |
| Laureth-9 | | 2,00 |
| Poloxamer-184 | | 2,00 |
| eau purifiée | q.s.p. | 100,00 |

### Exemple 4

On prépare une émulsion huile-dans-eau (crème) ayant la composition pondérale suivante.

| | | |
|---|---|---|
| phytosphingosine | | 0,25 |
| nicotinamide (vitamine PP) | | 3,50 |
| émulsionnant (stéarate de glycéryle / Ceteareth 20 / Ceteareth 10 / alcool cétéarylique / palmitate de cétyle) | | 7,00 |
| alcool cétylique | | 1,00 |
| C12-13 alkyl lactate | | 10,00 |
| cyclomethicone / dimethiconol | | 1,00 |
| gomme de xanthane | | 0,50 |
| butylène glycol | | 5,00 |
| conservateurs | | 0,50 |
| eau purifiée | q.s.p. | 100,00 |

### Exemple 5

Un shampooing conforme à la présente invention est préparé suivant les techniques classiques, et sa composition pondérale est la suivante.

| | | |
|---|---|---|
| Chlorhydrate de phytosphingosine | | 0,10 |
| nicotinamide (vitamine PP) | | 2,00 |
| émulsionnant (Sepiperl N®) | | 2,50 |
| cyclométhicone | | 2,50 |
| épaississant (Aculyn 22 / 33®) | | 6,50 |
| triéthanolamine | | 1,10 |
| base lavante (tensioactifs) | | 64,00 |
| conservateurs | | 0,20 |
| eau purifiée | q.s.p. | 100,00 |

### Exemple 6

Suivant les techniques classiques, on prépare une crème (émulsion huile dans eau) ayant la composition suivante :

| | | |
|---|---|---|
| phytosphingosine | | 0,20 |
| nicotinamide (vitamine PP) | | 3,00 |
| ciclopirox | | 1,00 |
| émulsionnant (stéarate de glycéryle / Ceteareth 20 / Ceteareth 10 / alcool cétéarylique / palmitate de cétyle) | | 7,00 |
| alcool cétylique | | 1,00 |
| C12-13 alkyl lactate | | 10,00 |
| cyclomethicone | | 1,00 |
| butylène glycol | | 5,00 |
| conservateurs | | 0,50 |
| eau purifiée | q.s.p. | 100,00 |

### Exemple 7

On prépare un shampooing ayant la composition suivante par les méthodes usuelles de la technique :

| | | |
|---|---|---|
| phytosphingosine (chlorhydrate) | | 0,20 |
| nicotinamide (vitamine PP) | | 3,50 |
| ciclopiroxolamine | | 1,50 |
| laureth sulfate de sodium | | 5,00 |
| émulsionnant (Montanov S) | | 2,50 |
| tensioactif non ionique | | 5,00 |
| tensioactif amphotère | | 28,00 |
| épaississant (Aculyn) | | 4,00 |
| cyclomethicone | | 2,50 |
| conditionneur | | 0,80 |
| triéthylamine | | 4,00 |
| conservateurs | | 0,20 |
| eau déminéralisée | q.s.p. | 100,00 |
| Le pH de la composition est 7,5. | | |

Cette composition peut être utilisée sous forme de shampooing une ou plusieurs fois par semaine.

### Exemple 8

Des essais cliniques ont été réalisés sur 10 patients (5 garçons et 5 filles) âgés de 14 à 23 ans pour mettre en évidence l'efficacité de la composition de l'invention dans le traitement de l'acné.

La composition utilisée est un gel conforme à celui dont la composition est indiquée dans l'Exemple 2.

Les 10 patients présentent une acné inflammatoire du visage avec atteinte du front, des joues, du nez et du menton. Un traitement classique au peroxyde de benzoyle (4 cas), à l'érythromycine (3 cas) et au gel de nicotinamide (3 cas) n'a apporté aucune amélioration notable.

Par contre, une amélioration notable a été observée dès le 8ème jour par un traitement au moyen du gel de l'Exemple 2, à raison de deux applications quotidiennes pendant un mois, dans des conditions de parfaite tolérance. Le traitement n'a provoqué aucun dessèchement malgré la présence d'alcool et l'amélioration de la qualité de la peau des patients s'est traduite par une peau plus claire, plus lisse, avec beaucoup moins de comédons.

Cinq autres patients présentant une folliculite à gram-négatif péribuccale induite par la prise de doxycycline par voie orale ont été traités avec succès par le même gel pendant trois semaines à raison de deux applications par jour.

Enfin, trois patients traités simultanément par l'isotrétinoïne à la dose de 0,5 mg/kg pendant 5 mois et une crème conforme à l'invention, dont la composition est donnée dans l'Exemple 4, n'ont présenté aucune surinfection staphylococcique tout au long du traitement. Les poussées inflammatoires en début de traitement ont été bien contrôlées, et la sécheresse du visage induite par le rétinoïde a été très atténuée.

### Exemple 9

Des essais cliniques ont été réalisés sur 10 patients (5 garçons et 5 filles) âgés de 6 mois à 30 ans pour mettre en évidence l'efficacité de la composition de l'invention dans le traitement de la dermite atopique.

La composition utilisée est une crème conforme à celle dont la composition est indiquée dans l'Exemple 4. Elle est appliquée pendant 6 semaines à raison de deux applications par jour.

On observe alors une diminution significative des populations de Staphylococcus aureus présentes sur la peau des patients. Une amélioration parallèle du prurit et des manifestations eczémateuses est observée dès la deuxième semaine de traitement, avec une diminution très sensible des besoins en corticoïdes locaux, ainsi qu'une réduction progressive de la sécheresse de la peau.

Ces améliorations peuvent s'expliquer par la restauration de l'effet barrière du stratum corneum par une synthèse accrue des céramides et des autres lipides du cément intercornéocytaire sous l'action conjointe et complémentaire de la phytosphingosine et de la vitamine PP.

5 autres patients âgés de 3 mois à 4 ans souffrant de dermite atopique au niveau du visage ont été traités par application d'une lotion micellaire conforme à l'Exemple 3 au moyen d'un coton imprégné de la lotion, deux à trois fois par jour pendant 5 semaines.

On observe alors une amélioration sensible et rapide de l'état des patients avec une tolérance nettement supérieure par comparaison avec les produits usuels, et un meilleur contrôle des phénomènes inflammatoires locaux.

Un traitement a été effectué sur 5 autres patients souffrant d'une forme particulière de dermite atopique touchant le cuir chevelu, le visage et le haut du tronc, liée au développement d'une surpopulation pytirosporique responsable de la réaction de sensibilisation cutanée. Le traitement consiste à appliquer conjointement pendant 4 semaines un shampooing conforme à l'exemple 5, à raison de deux fois par semaine, et une crème suivant l'Exemple 4 à raison de deux applications quotidiennes.

Une amélioration sensible de l'état des patients, sans réaction secondaire, a été observé dès la deuxième semaine du traitement.

### Exemple 10

Une étude bactériologique a été menée comme indiqué ci-après et a mis en évidence le pouvoir inhibiteur d'une composition suivant l'invention, conforme à celle décrite dans l'Exemple 1, sur trois souches bactériennes, par comparaison avec un témoin, une solution hydroglycolique, et une solution hydroglycolique contenant de la phytosphingosine mais ne contenant pas de vitamine PP.

Les souches bactériennes utilisées sont Propioni bacterium acnes, Staphylococcus aureus et Serratia marcescens. La première se retrouve habituellement dans la flore pathogène saprophyte, tandis que les deux autres sont généralement présentes lors de surinfections cutanées pathogènes. De plus, les essais ont aussi été effectués sur une souche levurique, Malassezia furfur, qui fait aussi partie de la flore saprophyte cutanée.

Le témoin actif utilisé est une solution d'ammonium quaternaire (Bactopin® ).

Le témoin hydroglycolique a la composition suivante :

| | | |
|---|---|---|
| Propylèneglycol | | 5,0 |
| Ethanol | | 54,9 |
| Eau | q.s.p. | 100,0 |

La solution hydroglycolique contenant de la phytosphingosine a la composition suivante :

| | | |
|---|---|---|
| Propylèneglycol | | 5,0 |
| Ethanol | | 54,9 |
| Chlorhydrate de phytosphingosine | | 0,2 |
| Eau | q.s.p. | 100,0 |

La solution de l'invention est une variante simplifiée de la composition de l'Exemple 1 :

| | | |
|---|---|---|
| Propylèneglycol | | 5,0 |
| Ethanol | | 54,9 |
| Chlorhydrate de phytosphingosine | | 0,2 |
| Vitamine PP | | 4,0 |
| Eau | q.s.p. | 100,0 |

Les compositions ci-dessus sont exprimées en parties en poids.

Les essais sont effectués en mesurant la zone d'inhibition de croissance des souches autour des puits contenant les produits à tester. Le pouvoir inhibiteur du produit est d'autant plus important, et par conséquent son efficacité plus grande sur les souches testées, que la zone est importante. Le pouvoir inhibiteur est nul dans le cas du témoin hydroglycolique qui ne contient que du propylène glycol, de l'éthanol et de l'eau, tandis qu'il est maximal dans le cas du témoin actif (solution d'ammonium quaternaire).

| souche | témoin | témoin actif | phytosphingosine | Phytosphingosine + vit. PP |
|---|---|---|---|---|
| Staphylococcus aureus | 0 | 22 | 17,3 | 22,0 |
| Serratia marcescens | 0 | 18 | 12,3 | 12,3 |
| Propionibacterium acnes | 0 | 22 | 10,3 | 15,3 |
| Malassezia furfur | 0 | 22 | 17,0 | 20,0 |

On constate alors que la solution conforme à l'invention présente un pouvoir inhibiteur sur les quatre souches, et que son efficacité est équivalente à celle du témoin hydroglycolique (témoin hydroglycolique) et nettement supérieure à celle de la solution hydroglycolique contenant de la phytosphingosine. De plus, l'activité antibactérienne et antilevurique de la composition de l'invention est potentialisée par l'addition de vitamine PP.

Au contraire, la solution de comparaison à 0,2% de chlorhydrate de phytosphingosine présente une activité antibactérienne inférieure à celle du témoin hydroglycolique.

Le témoin actif, constitué par une solution pure d'ammonium quaternaire, est utilisé dans ce test à titre de référence, mais il ne pourrait pas être utilisé sous cette forme en dermatologie.

## Revendications

1. Composition dermatologique utile pour le traitement des affections de la peau, **caractérisée en ce qu'**elle comprend en combinaison le nicotinamide (vitamine PP) et au moins une base sphingoïde choisie parmi la phytosphingosine, la tétracétylphytosphingosine, la N-acétylphytosphingosine, et le chlorhydrate de phytosphingosine.

2. Composition selon la revendication 1, **caractérisée en ce que** la teneur en nicotinamide, est comprise entre 0,1 et 15 % en poids par rapport au poids total de la composition.

3. Composition selon la revendication 2, **caractérisée en ce que** la teneur en nicotinamide, est comprise entre 1 et 10 % en poids par rapport au poids total de la composition.

4. Composition selon la revendication 1, **caractérisée en ce que** la teneur en base sphingoïde est comprise entre 0,01 % et 5 % par rapport au poids total de la composition.

5. Composition selon la revendication 4, **caractérisée en ce que** la teneur en base sphingoïde est comprise entre 0,05 % et 2 % par rapport au poids total de la composition.

6. Composition selon l'une quelconque des revendications précédentes, **caractérisée en ce qu'**elle comprend en outre du ciclopirox ou de la ciclopiroxolamine.

7. Composition selon la revendication 6, **caractérisée en ce que** la teneur en ciclopirox ou en ciclopiroxolamine est comprise entre 0 et 5 % en poids par rapport au poids total de la composition.

8. Composition selon l'une quelconque des revendications précédentes, **caractérisée en ce qu'**elle est associée à une ou plusieurs cyclines ou à l'isotrétinoïne.

9. Utilisation du nicotinamide (vitamine PP), et d'une base sphingoïde selon la revendication 1 pour la préparation d'un médicament pour le traitement de l'acné et de la dermite atopique.

## Claims

1. A dermatological composition useful for the treatment of skin conditions, **characterized in that** it comprises, in combination, nicotinamide (vitamin PP), and at least one sphingoid base selected from phytosphingosine, tetraacetylphytosphingosine, N-acetylphytosphingosine, and phytosphingosine hydrochloride.

2. The composition of claim 1, **characterized in that** the content of nicotinamide is between 0.1 and 15% by weight relative to the total weight of the composition.

3. The composition of claim 2, **characterized in that** the content of nicotinamide is between 1 and 10% by weight relative to the total weight of the composition.

4. The composition of claim 1, **characterized in that** the content of sphingoid base is between 0.01% and 5% relative to the total weight of the composition.

5. The composition of claim 4, **characterized in that** the content of sphingoid base is between 0.05% and 2% relative to the total weight of the composition.

6. The composition according to any one of the preceding claims, **characterized in that** it further comprises ciclopirox or ciclopiroxolamine.

7. The composition of claim 6, **characterized in that** the content of ciclopirox or of ciclopiroxolamine is between 0 and 5% by weight relative to the total weight of the composition.

8. The composition according to any one of the preceding claims, **characterized in that** it is combined with one or more cyclins or with isotretinoin.

9. The use of nicotinamide (vitamin PP), and of a sphingoid base according to claim 1, for preparing a medicinal product for the treatment of acne and of atopic dermatitis.

## Patentansprüche

1. Dermatologische Zusammensetzung, die zur Behandlung von Hautleiden dient, **dadurch gekennzeichnet, dass** sie eine Kombination aus Nicotinsäureamid (Vitamin PP) und zumindest einer Sphingo-Base, ausgewählt aus Phytosphingosin, Tetracetylphytosphingosin, N-Acetylphytosphingosin und Phytosphingosinchlorhydrat, umfasst.

2. Zusammensetzung nach Anspruch 1, **dadurch gekennzeichnet, dass** der Gehalt an Nicotinsäureamid zwischen 0,1 und 15 Gew.-%, bezogen auf das Gesamtgewicht der Zusammensetzung, beträgt.

3. Zusammensetzung nach Anspruch 2, **dadurch gekennzeichnet, dass** der Gehalt an Nicotinsäureamid zwischen 1 und 10 Gew.-%, bezogen auf das Gesamtgewicht der Zusammensetzung, beträgt.

4. Zusammensetzung nach Anspruch 1, **dadurch gekennzeichnet, dass** der Gehalt an Sphingo-Base zwischen 0,01 und 5 %, bezogen auf das Gesamtgewicht der Zusammensetzung, beträgt.

5. Zusammensetzung nach Anspruch 4, **dadurch gekennzeichnet, dass** der Gehalt an Sphingo-Base zwischen 0,05 und 2 %, bezogen auf das Gesamtgewicht der Zusammensetzung, beträgt.

6. Zusammensetzung nach einem der vorangehenden Ansprüche, **dadurch gekennzeichnet, dass** sie darüber hinaus Ciclopirox oder Ciclopiroxolamin umfasst.

7. Zusammensetzung nach Anspruch 6, **dadurch gekennzeichnet, dass** der Gehalt an Ciclopirox oder Ciclopiroxolamin zwischen 0 und 5 Gew.-%, bezogen auf das Gesamtgewicht der Zusammensetzung, beträgt.

8. Zusammensetzung nach einem der vorangehenden Ansprüche, **dadurch gekennzeichnet, dass** sie mit einem oder mehreren Cyclinen oder mit Isotretinoin assoziiert ist.

9. Verwendung von Nicotinsäureamid (Vitamin PP) und einer Sphingo-Base nach Anspruch 1 zur Herstellung eines Medikaments zur Behandlung von Akne und atopischer Dermatitis.
